# EUROPÄISCHE PATENTANMELDUNG

(11) **EP 1 886 684 A1**
(43) Veröffentlichungstag der Anmeldung: **13.02.2008**
(21) Anmeldenummer: 06016450.6
(22) Anmeldetag: 07.08.2006
(51) Int. Cl.: A61K 31/454, A61K 9/20

(54) **Rimonabant-haltiges Arzneimittel**

(71) Anmelder: Ratiopharm GmbH, 89079 Ulm (DE)
(72) Erfinder: Alles, Rainer, 81476 München (DE); Schulze Nahrup, Julia, 82061 Neuried (DE); Kubecek, Jiri, 90419 Nürnberg (DE); Kuttesch Edith, 80797 München (DE)
(74) Vertreter: Best, Michael

(57) **Zusammenfassung**

Erfindungsgemäß werden Arzneimittel zur oralen Verabreichung des Wirkstoffes Rimonabant zur Verfügung gestellt, bei denen der Wirkstoff mit einem Bindemittel, einem Sprengmittel und gegebenenfalls weiteren pharmazeutischen Hilfsstoffen durch trockenes Vermischen und gegebenenfalls durch Trockengranulation oder durch Direktverpressen unter Ausschluss eines Nassgranulationsverfahrens verarbeitet wird.

## Beschreibung

Die Erfindung betrifft ein neues Arzneimittel mit dem Wirkstoff 5-(4-Chlorphenyl)-1-(2,4-dichlorphenyl)-4-methyl-N-1-piperidinyl-1 H-pyrazol-3-carboxamid, der auch unter der Bezeichnung Rimonabant bekannt ist. Das Arzneimittel wird durch trockenes Vermischen und gegebenenfalls durch Direktverpressen (bzw. Direkttablettieren) oder Trockengranulationsverfahren hergestellt.

Die Verbindung 5-(4-Chlorphenyl)-1-(2,4-dichlorphenyl)-4-methyl-N-1-piperidinyl-1H-pyrazol-3-carboxamid ist ein bekannter Wirkstoff mit der chemischen Formel

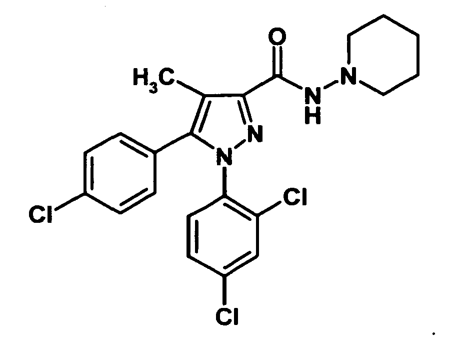

Für die Verbindung wird in älteren Druckschriften auch der Codename SR141716 verwendet. In der EP-A 969 832 wird Rimonabant noch als Verbindung A bezeichnet. Mittlerweile ist aber die Bezeichnung Rimonabant für diese Verbindung üblich und wird im Rahmen dieser Anmeldung verwendet.

Rimonabant ist ein selektiver Antagonist des Cannabinoidrezeptors CB1. Die Verbindung unterdrückt damit Hungergefühle und führt zu verringerter Nahrungsaufnahme sowie verändertem Zucker- und Lipidmetabolismus. Außerdem blockiert Rimonabant positive Effekte von Nikotin und anderen psychotropen Substanzen. Rimonabant ist ein Wirkstoff, der insbesondere für sogenannte "Lifestyle"-Medikamente verwendet werden kann, beispielsweise zur Behandlung von Fettleibigkeit und zur Unterstützung bei der Nikotinentwöhnung. Rimonabant wird ebenfalls als geeignet zur Triglycerid-/Lipidsenkung, zur Behandlung des metabolischen Syndroms, zur Behandlung der Typ-II-Diabetes und zur Behandlung der Arteriosklerose angesehen. Der Wirkstoff soll ebenfalls positive Effekte beim Lernen, hinsichtlich der Schmerzempfindlichkeit, bei Fettleber und bei der Parkinson-Erkrankung haben. Neuere Daten weisen auch auf potentielle Verwendung von Rimonabant-haltigen Arzneimitteln bei Tumoren hin (z.B. Brustkrebs, Lipom, Liposarkom). Zu den möglichen Indikationen kann auf die EP-A 656 354 und die EP-A 969 832 verwiesen werden.

Rimonabant ist generisch in der europäischen Patentanmeldung EP-A 576 357 und speziell in der europäischen Patentanmeldung EP-A 656 354 offenbart.

Rimonabant kann in mehreren Polymorphen Formen vorkommen, so offenbart die WO 2003/040105 das Polymorph II der Verbindung, nach dem Herstellungsverfahren der EP-A 656 354 erhält man die polymorphe Form I der Verbindung. Bezüglich der Herstellungsverfahren des Rimonabants kann auf die EP-A 656 354 und die WO 2003/040105 verwiesen werden.

Rimonabant ist eine hydrophobe Verbindung. Die Formulierung des Wirkstoffes zu festen oralen Arzneimitteln ist nicht problemlos, da nach oraler Verabreichung eine gute Freisetzung des Wirkstoffs sichergestellt sein muss. Gerade bei hydrophoben Verbindungen wie Rimonabant ist es schwierig, bei oraler Verabreichung eine gute Freisetzung im (wässrigen) gastrointestinalen Trakt zu gewährleisten.

Die am weitesten verbreiteten Wege zur Herstellung von festen oralen Arzneimitteln umfassen einen Nassgranulationsschritt. Solche Nassgranulationsverfahren werden auch im Stand der Technik für die Formulierung des Wirkstoffs Rimonabant vorgeschlagen. So setzt die EP-A 969 832 zur Herstellung von festen oralen Arzneimitteln mit dem Wirkstoff Rimonabant den Wirkstoff in mikronisierter Form ein und formuliert ihn mittels eines Nassgranulationsverfahrens zu einer festen oralen Dosierungsform, insbesondere einer Weichgelatinekapsel oder einer Tablette. Um eine gute Verarbeitbarkeit und Freisetzung zu gewährleisten, muss bei dem Nassgranulationsverfahren des Standes der Technik ein spezielles Tensid, nämlich ein Natriumalkylsulfat, und eine sehr hohe Menge an Sprengmittel (in der EP-A 969 832 im Bereich von 2,5 bis 10 Gew.-%) eingesetzt werden.

Die in der EP-A 969 832 offenbarten Rimonabant-haltigen Arzneimittel zeigen zwar eine gute Freisetzung des Wirkstoffs, sind aber aufwendig herzustellen. Soweit es sich bei diesen Arzneimitteln um Tabletten handelt, weisen sie auch eine relativ geringe Bruchfestigkeit auf.

Aufgabe der vorliegenden Erfindung ist es daher, oral zu verabreichende feste Arzneimittel mit dem Wirkstoff Rimonabant zur Verfügung zu stellen, die eine Freisetzung zeigen, die mindestens so gut ist wie die der oralen Arzneimittel des Standes der Technik, wie sie beispielsweise in der EP-A 969 832 beschrieben werden. Diese Arzneimittel sollten aber einfacher herzustellen sein, als die aus der EP-A 969 832 bekannten Arzneimittel und, soweit es sich um Tabletten handelt, sollten sie eine bessere Bruchfestigkeit zeigen als die bekannten Tabletten.

Diese Aufgabe wird durch den Gegenstand der Patentansprüche gelöst.

Überraschenderweise wurde erfindungsgemäß gefunden, dass die im Stand der Technik beschriebenen Schwierigkeiten bei der Freisetzung des Rimonabants aus festen Arzneimitteln nicht auftreten, wenn bei der Herstellung der Arzneimittel kein Nassgranulationsverfahren eingesetzt wird, sondern die Bestandteile des Arzneimittels trocken vermischt werden. Gegebenenfalls kann auch eine Trockengranulation erfolgen und, insbesondere bei der Herstellung von Tabletten, eine Direkttablettierung (d.h. eine Verarbeitung des trockenen Gemisches durch Direktverpressen zu Tabletten). Die erfindungsgemäß hergestellten Arzneimittel, die durch einfaches trockenes Vermischen der Bestandteile des Arzneimittels hergestellt werden können, zeigen ausgezeichnete Freisetzungsprofile, die die gesetzlichen Anforderungen erfüllen, vergleichbar der Freisetzungsprofile der nach dem Nassgranulationsverfahren des Standes der Technik gemäß der EP-A 969 832 hergestellten Arzneimittel. Es ist dabei nicht erforderlich, dass ein sehr hoher Gehalt an Sprengmitteln eingesetzt wird, wie er in der EP-A 969 832 für das Nassgranulationsverfahren als erforderlich angesehen wird, und auch die Verwendung eines Alkylsulfats als Netzmittel bzw. Tensid ist bei den erfindungsgemäßen Arzneimitteln nicht zwingend, um die gewünschte hohe Freisetzung des Wirkstoffs aus den Arzneimitteln zu gewährleisten. Dadurch, dass auf ein Nassgranulationsverfahren verzichtet wird, ergeben sich auch verfahrenstechnische Vorteile. Offensichtlich hat die Nassgranulation negative Auswirkungen auf die Freisetzung des Wirkstoffs, die durch den hohen Anteil an Sprengmittel und ein spezielles Tensid ausgeglichen werden müssen. Diese negativen Auswirkungen können durch eine trockene Verarbeitung der Inhaltsstoffe ohne Nassgranulation vermieden werden.

Schließlich wurde überraschend gefunden, dass bei Tabletten, die nicht durch ein Nassgranulationsverfahren hergestellt wurden, sondern erfindungsgemäß durch trockenes Vermischen der Bestandteile und Direktverpressen, eine bessere Tablettenhärte erzielt wird als bei den in der EP-A 969 832 beispielhaft offenbarten Tabletten.

Die erfindungsgemäßen Arzneimittel enthalten als Wirkstoff die Verbindung Rimonabant, ein Salz dieser Verbindung, ein Solvat der Verbindung oder ein Solvat eines Salzes dieser Verbindung. Besonders bevorzugt wird als Wirkstoff das Rimonabant in Basenform oder in Form des Hydrochloridsalzes eingesetzt. Weitere bevorzugte Salze des Rimonabants sind das Methansulfonat, das Hemifumarat, das p-Toluolsulfonat, das Hydrogensulfat, das Dihydrogenphosphat, das Hydrobromid, das Sulfat, das Methylsulfat, das Oxalat, das Maleat, das Fumarat, das 2-Naphthalinsulfonat, das Glykonat, das Gluconat, das Citrat und das Isethionat des Rimonabants. Besonders bevorzugte Solvate sind das Ethanolsolvat und das Aceton-Hemisolvat. Besonders bevorzugt ist das Ethanolsolvat des Hydrochloridsalzes des Rimonabants und das Ethanolsolvat des Rimonabants in Basenform. Insbesondere das Methansulfonat kann auch als Aceton-Hemisolvat vorliegen.

Soweit in der folgenden Beschreibung auf Rimonabant oder den "Wirkstoff" Bezug genommen wird, soll jeweils Rimonabant in Form der freien Base oder in Form eines Salzes oder Solvats oder eines Solvats des Salzes wie vorstehend verstanden werden.

Der Anteil des Wirkstoffs in dem erfindungsgemäßen Arzneimittel beträgt in der Regel 0,5 bis 50 Gew.-%, stärker bevorzugt 1 bis 30 Gew.-%, noch stärker bevorzugt 1 bis 20 Gew.-%, insbesondere 5 bis 20 Gew.-% und am stärksten bevorzugt 10 bis 20 Gew.-%. Eine übliche Dosiseinheit des erfindungsgemäßen Arzneimittels enthält damit bevorzugt 2 mg bis 100 mg, stärker bevorzugt 2 mg bis 60 mg, insbesondere 15 mg bis 40 mg, z.B. 10, 15, 20, 25 oder 30 mg des Wirkstoffs.

Im Rahmen dieser Anmeldung beziehen sich prozentuale Angaben und Anteile immer auf das Gewicht des Arzneimittels, sofern nichts anderes ausdrücklich angegeben oder aufgrund der Umstände ersichtlich ist. Wenn von einer Menge des Wirkstoffs gesprochen wird, ist hierunter im Einklang mit den vorstehenden Ausführungen die Menge des Rimonabants als Base zu verstehen, sofern das Rimonabant als Base eingesetzt wird. Sofern das Rimonabant als Salz oder Solvat oder Solvat eines Salzes eingesetzt wird, ist hierunter die Menge an dem Salz, dem Solvat oder dem Solvat des Salzes zu verstehen.

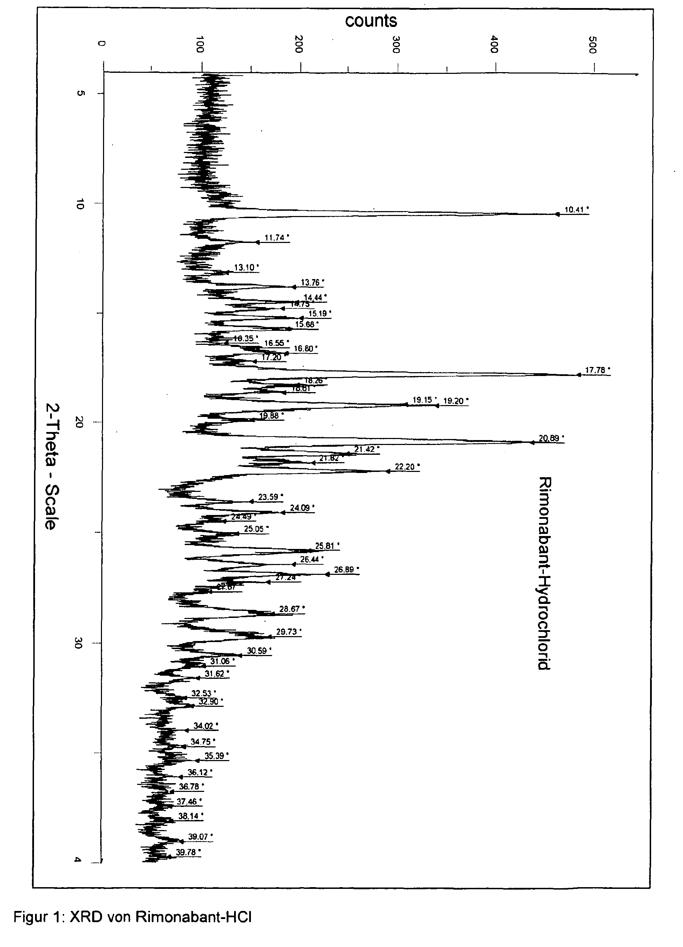

Rimonabant kann in verschiedenen polymorphen Formen vorliegen. Erfindungsgemäß kann jede polymorphe Form, jede amorphe Form und auch Gemische der polymorphen Formen miteinander und mit einer amorphen Form verwendet werden. Erfindungsgemäß besonders bevorzugt wird die polymorphe Form I, die sich bei dem Verfahren der EP-A 656 354 ergibt. Ebenfalls bevorzugt ist das in WO-2003/040105 beschriebene Polymorph II. Ein Pulverdiffraktogramm des nach dem Beispiel 3 der EP-A 656 354 hergestellten Hydrochloridsalzes des Rimonabants ist als Figur 1 beigefügt. Diese Form des Wirkstoffes Rimonabant ist ebenfalls erfindungsgemäß bevorzugt.

Erfindungsgemäß bevorzugt wird das Rimonabant in mikronisierter Form eingesetzt. Die Mikronisierung kann auf übliche Art und Weise erfolgen, beispielsweise mit einer Luftstrahlmühle. Die so mikronisierten Wirkstoffteilchen können beispielsweise durch Ultraschallbehandlung homogenisiert werden. Während für die Nassgranulationsverfahren des Standes der Technik sehr kleine Wirkstoffteilchen eingesetzt werden müssen, können für die erfindungsgemäßen Arzneimittel größere Wirkstoffpartikel verwendet werden. Insbesondere für die Herstellung von Tabletten durch Direktverpressen kann es auch vorteilhaft sein, größere Wirkstoffteilchen zu verwenden, da hierdurch die Fließfähigkeit und Verarbeitbarkeit des Gemisches bei der Direktverpressung verbessert wird. Hierin liegt ein weiterer Vorteil der Erfindung, da im allgemeinen der wirtschaftliche und technische Aufwand zur Herstellung größerer Partikel geringer ist, als der Aufwand zur Gewinnung sehr kleiner Partikel.

Erfindungsgemäß bevorzugt beträgt der Teilchendurchmesser von 50% der verwendeten Wirkstoffteilchen (D₅₀) 2 µM oder darüber, stärker bevorzugt 2,5 µM oder darüber und insbesondere etwa 3 µM. Die Größe von 80% der Wirkstoffteilchen (D₈₀) sollte vorteilhafterweise 80 µM nicht überschreiten, bevorzugt sind 50% der Teilchen jedoch nicht größer als 10 µM und insbesondere nicht größer als 6 µM. Besonders bevorzugt ist der D₅₀-Wert der Wirkstoffteilchen in dem erfindungsgemäßen Arzneimittel daher im Bereich von 2,5 µM bis 6 µM, insbesondere liegt er bei etwa 3 µM. Innerhalb dieser Bereiche erhält man einerseits eine sehr gute Freisetzung, andererseits eine sehr gute Verarbeitbarkeit, insbesondere bei der Direkttablettierung.

Neben dem Wirkstoff enthält das erfindungsgemäße Arzneimittel noch mindestens ein Bindemittel und mindestens ein Sprengmittel.

Die geeigneten Bindemittel sind nicht besonders eingeschränkt und es können übliche, im Stand der Technik bekannte Bindemittel verwendet werden. Besonders bevorzugte Bindemittel sind Copovidon, Povidon, Alginsäure und Alginate (wie Natriumalginat), Cellulose und Cellulosederivate (wie Natriumcarboxymethylcellulose, Ethylcellulose, Hydroxyethylcellulose, Hydroxypropylcellulose, Hydroxypropylmethylcellulose oder Methylcellulose und niedrigsubstituierte Hydroxypropylcellulose), Gelatine und Acrylsäurepolymere. Besonders bevorzugt ist das Bindemittel Copovidon, und hier kann beispielsweise das kommerzielle Produkt Kollidon VA 64 genannt werden. Unter den Povidonen kann beispielsweise das kommerzielle Produkt Povidon K 30 genannt werden.

Der Gehalt an Bindemittel in dem erfindungsgemäßen Arzneimittel ist nicht besonders eingeschränkt und kann von einem Fachmann leicht in Abhängigkeit des speziellen Arzneimittels gewählt werden. Üblicherweise ist der Anteil des Bindemittels im Bereich von 0,5 bis 10%, bevorzugt im Bereich von 0,5 bis 5%, insbesondere im Bereich von 1 bis 5%.

Auch das verwendbare Sprengmittel ist nicht besonders eingeschränkt. Hier können insbesondere Alginate, Polysaccharide, Caseine, anorganische Zerfallshilfsmittel wie z.B. Bentonite, aber insbesondere auch Cellulose und Cellulosederivate genannt werden wie Natriumcarboxymethylcellulose, vernetzte Natriumcarboxymethylcellulose, Crospovidon, vorgelatinisierte Stärke und Natriumcarboxymethylstärke. Besonders bevorzugt ist als Sprengmittel Natriumcarboxymethylstärke, beispielsweise die unter der Bezeichnung Explotab erhältlichen Produkte.

Anders als bei den im Stand der Technik durch Nassgranulation hergestellten Arzneimitteln ist es bei den erfindungsgemäßen Arzneimitteln nicht erforderlich, eine sehr hohe Menge an Sprengmittel zu verwenden. So ist die Menge an Sprengmittel bevorzugt geringer als 4%, stärker bevorzugt geringer als 3%, am stärksten bevorzugt geringer als 2,5%, z.B. etwa 2%. Allerdings sind in der Regel zumindest 0,1%, stärker bevorzugt zumindest 0,5% und am stärksten bevorzugt zumindest 1 % Sprengmittel vorhanden.

Das erfindungsgemäße Arzneimittel enthält in der Regel noch weitere pharmazeutische Hilfsstoffe, wie Füllstoffe, insbesondere anorganische Calciumverbindungen, wobei Calciumhydrogenphosphat besonders bevorzugt ist. Auch andere Calciumphosphate können verwendet werden. Geeignete Füllstoffe sind auch Zucker wie Lactose oder Mannit und bekannte Cellulose und Cellulosederivate, insbesondere mikrokristalline Cellulose, aber auch Stärke, Maisstärke oder vorgelatinisierte Stärke und Cyclodextrine. Besonders bevorzugt sind auch Gemische aus mehreren Füllstoffen, beispielsweise ein Gemisch aus einer anorganischen Calciumverbindung wie Calciumphosphat oder Calciumhydrogenphosphat und mikrokristalliner Cellulose. Die Füllstoffe können insbesondere auch ausgewählt werden, um die Verarbeitungseigenschaften der erfindungsgemäßen Arzneimittel zu verbessern. So kann eine grobkörnigere mikrokristalline Cellulose verwendet werden, um die Fließeigenschaften des Gemisches zu verbessern, was insbesondere bei einer Direkttablettierung (bzw. der Herstellung einer Tablette durch Direktverpressen) von Vorteil sein kann. Als mikrokristalline Cellulose werden bevorzugt mikrokristalline Cellulosen vom Avicel-Typ wie z.B. Avicel PH 101 oder Avicel PH 200 verwendet. Für Direkttablettierung bzw. das Herstellen von Tabletten mit Direktverpressen sind insbesondere Avicel PH 200 oder größere mikrokristalline Cellulosetypen geeignet, insbesondere in Kombination mit anorganischen Calciumverbindungen.

Die erfindungsgemäßen Arzneimittel können auch Tenside bzw. Netzmittel enthalten. Anders als bei den Arzneimitteln des Standes der Technik, die durch Nassgranulation hergestellt werden, ist es aber nicht unbedingt erforderlich, ein Netzmittel einzusetzen. Insbesondere muss nicht notwendigerweise ein Alkylsulfat wie das in der EP-A 969 832 besonders bevorzugte Natriumdodecylsulfat eingesetzt werden. Falls die erfindungsgemäßen Arzneimittel daher Tenside bzw. Netzmittel enthalten, sind diese bevorzugt von Alkylsulfaten verschieden und insbesondere handelt es sich nicht um Natriumalkylsulfate wie Natriumdodecylsulfat. Bevorzugte Netzmittel sind Salze der Gallensäuren, Acylsarkosine, Polysorbate, Polyoxyethylenfettalkoholether, Polyethylenglykole und synthetische Blockcopolymere aus Ethylenoxid und Propylenoxid wie das bekannte Poloxamer. Poloxamer ist besonders bevorzugt. Selbstverständlich können auch Gemische mehrerer Verbindungen eingesetzt werden.

Falls Tenside bzw. Netzmittel vorhanden sind, sind sie in den üblicherweise verwendeten Mengen vorhanden, z.B. in einer Menge von 0,01 bis 1%, insbesondere von 0,05 bis 0,5%.

Die erfindungsgemäßen Arzneimittel können auch noch weitere übliche pharmazeutische Hilfsstoffe enthalten, wie sie z.B. in der EP-A 969 832 offenbart sind, beispielsweise Gleitmitte wie Fettsäuren oder Fettsäurederivate wie Calciumstearat, Glycerylmonostearat, Glycerylpalmitostearat, Magnesiumstearat, Natriumstearylfumarat, Zinkstearat, Stearinsäure, hydrierte Pflanzenöle wie hydriertes Rhizinusöl, Polyethylenglykole wie Polyethylenglykol, Natriumbenzoat, Talk, pyrogen gewonnene Kieselsäuren, hochdisperses Siliciumdioxid, entfettetes Milchpulver oder Paraffin. Die Gleitmittel sind in den erfindungsgemäßen pharmazeutischen Zusammensetzungen, falls sie vorhanden sind, in den üblichen Mengen von 0,2 bis 5%, insbesondere von 0,2 bis 3% vorhanden.

Weitere übliche Hilfsstoffe, die in den erfindungsgemäßen Arzneimitteln vorhanden sein können, sind beispielsweise Trennmittel wie Silicium-haltige Verbindungen wie Kieselerde oder Talk (beispielsweise in einem Gewichtsanteil von 0% bis 5% sowie Fließmittel wie wasserfreie kolloidale Kieselerde oder Kieselhydrogel, die beispielsweise in einer Menge von 0% bis 15% vorliegen können.

Weitere übliche pharmazeutische Hilfsstoffe umfassen Farbstoffe, Geschmacksstoffe etc.

Außerdem können erfindungsgemäß erhaltene Arzneimittel überzogen werden, z.B. zur Herstellung von Film- oder Lacktabletten bzw. Dragees. Bevorzugte Überzüge enthalten Zucker, Polymere (z.B. Polymethacrylate) oder Cellulosederivate und ggf. weitere Hilfsstoffe.

Die erfindungsgemäßen Arzneimittel können auf an sich bekannte Art und Weise hergestellt werden. Beispielsweise können die trockenen Verbindungen durch einfaches Vermischen in einem üblichen Trockenmischer (z.B. Freifallmischer Glatt CML10) und anschließendes Abfüllen des trockenen Gemisches in Hartgelatinekapseln, Sachets, etc. hergestellt werden. Alternativ dazu kann das Gemisch auch auf übliche Art und Weise einer Trockengranulation unterworfen werden und das Granulat kann ebenfalls entweder in Hartgelatinekapseln oder Sachets abgefüllt oder zu Tabletten verpresst werden. Neben der Abfüllung in Hartgelatinekapseln kann das Pulver bzw. das Granulat natürlich auch in andere übliche Behältnisse abgefüllt oder auf bekannte Art und Weise zu Weichgelatinekapseln verarbeitet werden.

Besonders vorteilhaft können gemäß der Erfindung aber Tabletten durch Direktverpressen hergestellt werden. Entsprechende Direkttablettierungsmethoden sind im Stand der Technik bekannt und hier kann beispielsweise auf das Standardwerk "Die Tablette" von Ritschel und Bauer-Brandl, Edition Cantor Verlag 2002 verwiesen werden. Die Herstellung der erfindungsgemäßen Arzneimittel wird auch in den folgenden Beispielen erläutert.

Die erfindungsgemäßen Tabletten haben überraschenderweise eine bessere Bruchfestigkeit als die Tabletten, die durch Nassgranulation gemäß den Beispielen der EP-A 969 832 erhalten werden. Erfindungsgemäß bevorzugt sind Tabletten mit einer Bruchfestigkeit von mindestens 95 N, stärker bevorzugt von mindestens 100 N. Die Bruchfestigkeit kann durch übliche Art und Weise nach Pharm. Eur. 2.9.8 bestimmt werden.

Erfindungsgemäß besonders bevorzugt sind Arzneimittel die folgende Bestandteile enthalten:
a) Rimonabant
   Mikrokristalline Cellulose
   Calciumhydrogenphosphat
   NA-Carboxymethylstärke
   Copovidon
   Gleitmittel
b) Rimonabant
   Mikrokristalline Cellulose
   Lactose monohydrat
   NA-Carboxymethylstärke
   Copovidon
   Gleitmittel
c) Rimonabant
   Mikrokristalline Cellulose
   Calciumhydrogenphosphat
   NA-Carboxymethylstärke
   Copovidon
   Polysorbat
   Gleitmittel
d) Tablettenkern wie Beispiel a)
   Filmüberzug: Polymethacrylat; Polyethylenglycol, Talkum

Die folgenden Beispiele erläutern die Erfindung.

### Beispiel 1 Herstellung des Wirkstoffs

5-(4-Chlorphenyl)-1-(2,4-dichlorphenyl)-4-methyl-N-1-piperidinyl-1H-pyrazol-3-carboxamid ("freie Base") wurde nach der EP-A 969 832 Beispiel 1 synthetisiert. Charakterisierung durch ¹H-NMR, IR, Schmelzpunkt (155.7°C), HPLC, XRD. Man erhielt das Polymorph I wie es in der WO03/040105 definiert wird.

5-(4-Chlorphenyl)-1-(2,4-dichlorphenyl)-4-methyl-N-1-piperidinyl-1H-pyrazol-3-carboxamid-chlorhydrat ("HCl-Salz") wurde nach der EP-A 969 832 Beispiel 3 synthetisiert. Charakterisierung durch ¹H-NMR, IR, Schmelzpunkt (227.1°C), Chlorid-Gehalt 6.94 ± 0.72%. Das Röntgendiffraktogramm ist in Figur 1 gezeigt.

Die Körngrößenverteilungskurve wurde in drei unabhängigen Messungen mittels Laserdiffraktomerie (Mastersizer Microplus, Malvern Instruments) aufgenommen. Nachfolgend wird die Korngröße jeweils mit folgenden Perzentilwerten angegeben: ein oberer Schnitt (D₈₀), der mittlere Teilchendurchmesser (D₅₀) und ein unterer Schnitt (D₁₀). Die Mikronisierung wurde durchgeführt mit einer Luftstrahlmühle (MC Jetmill® 50) für 60 - 65 sec bei 2.0 bar Stickstoff.

Die synthetisierte Base wies heterogene Aggregate auf (D₁₀ 1.7 µM; D₅₀ 10.2 µM; D₈₀ 30.3 µM). Durch Aufschlämmung in Wasser und Ultraschallbehandlung konnte ein homogenes Pulver erhalten werden (D₁₀ 1.3 µM; D₅₀ 6.0 µM; D₈₀ 14.2 µM).

Das synthetisierte Hydrochlorid-Salz war sehr heterogen mit großen Aggregaten (D₁₀ 1.1 µM; D₅₀ 33.7 µM; D₈₀ 81.3 µM). Durch Aufschlämmung in Wasser und Ultraschallbehandlung konnte ebenfalls ein homogenes Pulver erhalten werden (D₁₀ 0.5 µM; D₅₀ 1.2 µM; D₈₀ 10.0 µM).

XRDs nach Mikronisierung zeigen, dass das Polymorph und Kristallinität erhalten bleiben.

Für die Herstellung der Arzneimittel des Beispiels 2 wurden Wirkstoffteilchen hergestellt, deren Größenverteilung wie folgt war. Mittlerer Teilchendurchmesser (D₅₀) nach Mikronisierung:
- Base 3.3 µM (D₁₀ 0.9 µM; D₈₀ 7.4 µM).
- HCl-Salz 2.9 µM (D₁₀ 1.0 µM; D₈₀ 4.9 µM).

### Beispiel 2 Zusammensetzung der Arzneimittel

Gemäß der folgenden Tabelle wurden fünf Gemische mit dem Wirkstoff und pharmazeutischen Zusatzstoffen hergestellt. Das Gemisch A entspricht der Formulierung F aus der EP-A 969 832 (Tabelle 6). Gemisch B ist analog zusammengesetzt, enthält aber Rimonabant Hydrochlorid statt der freien Base des Wirkstoffs. Beide Gemische A und B wurden über Nassgranulation weiterverarbeitet. Die Gemische C, D und E entsprechen den erfindungsgemäßen Arzneimitteln, die ohne Nassgranulation nur über trockene Mischverfahren weiterverarbeitet wurden.

| **Zusammensetzung** | **A** | **B** | **C** | **D** | **E** |
|---|---|---|---|---|---|
| **Komponente** *Funktion* | **[%]** | **[%]** | **[%]** | **[%]** | **[%]** |
| Rimonabant Base *Wirkstoff* | 16.8 | | 16.7 | | 16.7 |
| Rimonabant-HCl *Wirkstoff* | | 17.9 | | 17.8 | |
| Amidon DE (Maisstärke) *Füllstoff* | 28.5 | 28.2 | | | |
| Avicel PH 101 (MCC) *Füllstoff* | | | 50.3 | 49.6 | |
| Avicel PH 200 *Füllstoff* | | | | | 50.3 |
| Granulac 200 (Lactosemonohydrat) *Füllstoff* | 46.4 | 45.8 | | | |
| DiCafos P (Calciumhydrogenphosphat) *Füllstoff* | | | 27.8 | 27.4 | |
| DiCafos (Calciumhydrogenphosphat) *Füllstoff* | | | | | 27.8 |
| Ac-Di-Sol (Na-Croscarmelose) *Sprengmittel* | 4.8 | 4.7 | | | |
| Explotab (Na-Carboxymethylstärke) *Sprengmittel* | | | 1.9 | 1.9 | 1.9 |
| Kollidon 30 (Povidon K 30) *Haft-* / *Bindemittel* | 2.4 | 2.4 | | | |
| Kollidon VA 64 (Copovidon) *Haft-*/ *Bindemittel* | | | 2.4 | 2.4 | 2.4 |
| Natriumdodecylsulfat *Netzmittel* | 0.1 | 0.1 | | | |
| gereinigtes Wasser *Granulationsflüssigkeit* | q.s. | q.s. | | | |
| Magnesiumstearat *Gleitmittel* | 1.0 | 0.9 | 1.0 | 1.0 | 1.0 |
| **Summe** | **100.0** | **100.0** | **100.0** | **100.0** | **100.0** |

Die Formulierungen A und B wurden durch Nassgranulation hergestellt. Die Nassgranulation erfolgte mit gereinigtem Wasser für 4 Min. Anschließend wurde 6 h bei 50°C getrocknet und es wurde auf 1000 µM gesiebt. Das Granulat wurde 10 Min. bei 45 upm mit Magnesiumstearat gemischt und in Hartgelatinekapseln abgefüllt.

Die Formulierungen C und D wurden durch einfaches Vermischen der Bestandteile für 4 Min. im Mörser hergestellt und das Arzneimittel wurde anschließend ebenfalls in Hartgelatinekapseln abgefüllt. Jede Hartgelatinekapsel enthielt etwa 180 mg Wirkstoff.

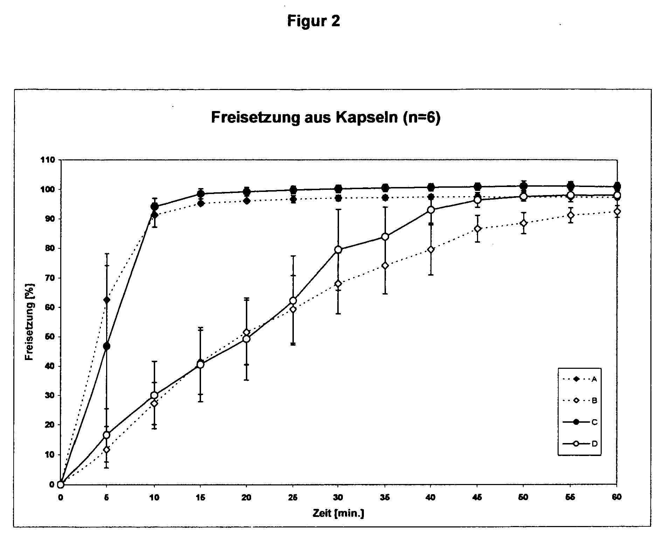

Die so erhaltenen Kapseln wurden auf die Freisetzung des Wirkstoffes nach dem in der EP-A 969 832 beschriebenen Verfahren getestet. Als Medium wurden 1000 ml ± 10 ml 0,1 normaler Citrat-Phosphat-Puffer mit 0,2% SDS bei einem pH von 3,0 und 37°C verwendet. Es wurde eine Standard-Paddel-Apparatur (USP-App. II, Distek Premiere 5100) bei 100 upm verwendet. Die Freisetzung des Rimonabants wurde per UV-Messung bei 276 nm gemessen. Das Ergebnis ist als Figur 2 gezeigt.

### Beispiel 3 Herstellung von Tabletten

Aus einem Granulat, das über Nassgranulation wie in Beispiel 2 beschrieben hergestellt wurde, wurden aus den Formulierungen A und B mit einer Korsch EK0-Presse runde facettierte Tabletten mit 8,0 mm Durchmesser hergestellt. Der Wirkstoffgehalt betrug jeweils etwa 30 mg. Aus den Bestandteilen der in Beispiel 2 gezeigten Formulierung E wurden durch Direktverpressen Tabletten hergestellt. Hierzu wurden alle Komponenten mit Ausnahme des Magnesiumstearats durch ein 500 µM-Sieb gesiebt und 20 Min. gemischt. Nach Zugabe von Magnesiumstearat (ebenfalls durch das 500 µM-Sieb gesiebt) wurde weitere 5 Min. gemischt und das Gemisch wurde ebenfalls auf einer Korsch EK0-Presse zu Tabletten mit etwa 30 mg Wirkstoff und einem Durchmesser von 8,0 mm verpresst.

Die Bruchfestigkeit der Tabletten wurde bestimmt und ist in folgender Tabelle zusammengefasst.

| | **A** | **B** | **E** |
|---|---|---|---|
| Kompressionskraft [kN] | ∼ 5.8 | ∼ 5.8 | ∼ 5.9 |
| Kompressionsdruck [mPa] | ∼ 110.0 | ∼ 110.0 | ∼ 110.0 |
| Tablettengewicht* [mg] | 179.3 | 181.7 | 180.3 |
| Bruchfestigkeit* [N] | 86.9 | 69.5 | 104.9 |
| Tablettenhöhe* [mm] | 2.80 | 2.79 | 2.56 |

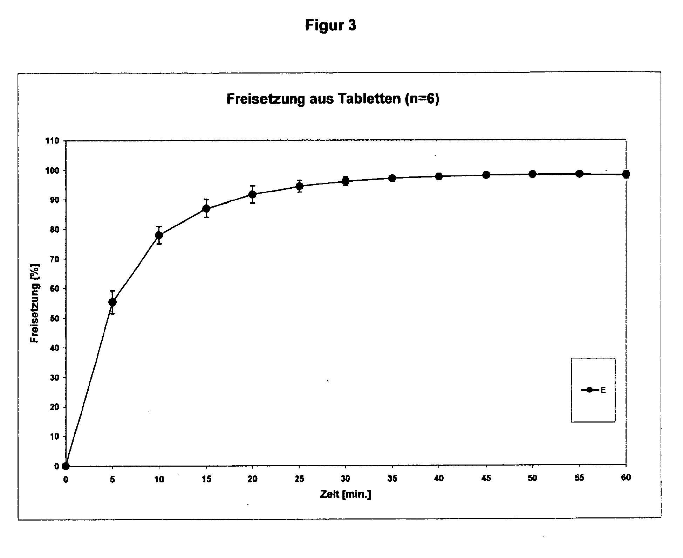

Die Freisetzung des Wirkstoffs aus der erfindungsgemäßen Tablette E erfüllt die pharmazeutischen Anforderungen an eine schnellfreisetzende Tablette (85% Freisetzung innerhalb von 15 Min.) (Figur 3).

## Patentansprüche

1. Arzneimittel zur oralen Verabreichung des Wirkstoffs 5-(4-Chlorphenyl)-1-(2,4-dichlorphenyl)-4-methyl-N-1-piperidinyl-1 H-pyrazol-3-carboxamid oder eines Salzes oder Solvats davon oder eines Solvats des Salzes des Wirkstoffs, erhältlich durch trockenes Vermischen und gegebenenfalls durch Trockengranulation oder Direktverpressen, unter Ausschluss eines Nassgranulationsverfahrens, von 0,5 bis 50 Gew.-% des Wirkstoffs oder des Salzes oder Solvats davon oder des Solvats des Salzes des Wirkstoffs, mindestens eines Bindemittels, mindestens eines Sprengmittels und gegebenenfalls weiterer pharmazeutischer Hilfsstoffe.

2. Arzneimittel nach Anspruch 1, **dadurch gekennzeichnet, dass** der Wirkstoff in Form des Hydrochloridsalzes oder eines Solvats des Hydrochloridsalzes vorliegt.

3. Arzneimittel nach Anspruch 1 oder 2, **dadurch gekennzeichnet, dass** es entweder kein Netzmittel enthält oder ein von einem Alkylsulfat verschiedenes Netzmittel enthält.

4. Arzneimittel nach Anspruch 3, **dadurch gekennzeichnet, dass** es ein oder mehrere Netzmittel enthält, ausgewählt aus Salzen der Gallensäuren, Acylsarkosinen, Polysorbaten, Polyoxyethylenfettalkoholethern, Polyethylenglykolen und synthetischen Blockcopolymeren aus Ethylenoxid und Propylenoxid.

5. Arzneimittel nach einem der Ansprüche 1 bis 4, **dadurch gekennzeichnet, dass** der Wirkstoff oder das Salz oder das Solvat davon oder das Solvat des Salzes des Wirkstoffs in mikronisierter Form vorliegt.

6. Arzneimittel nach Anspruch 5, **dadurch gekennzeichnet, dass** die mittlere Korngröße des Wirkstoffs oder des Salzes oder Solvats davon oder des Solvats des Salzes des Wirkstoffs 2,5 µm oder darüber ist.

7. Arzneimittel nach einem der Ansprüche 1 bis 6, **dadurch gekennzeichnet, dass** der Gehalt an Sprengmittel im Bereich von 0,1 bis 2 Gew.-% liegt, bezogen auf das Gesamtgewicht des Arzneimittels.

8. Arzneimittel nach einem der Ansprüche 1 bis 7, **dadurch gekennzeichnet, dass** es sich um eine Tablette handelt.

9. Arzneimittel nach Anspruch 8, **dadurch gekennzeichnet, dass** es sich um eine durch Direktverpressen hergestellte Tablette handelt.

10. Arzneimittel nach Anspruch 9, **dadurch gekennzeichnet, dass** die Tablette eine Bruchfestigkeit von zumindest 95 N aufweist.

11. Arzneimittel nach einem der Ansprüche 1 bis 7, **dadurch gekennzeichnet, dass** es sich um eine Hartgelatinekapsel handelt, die das Gemisch als Pulver oder Granulat enthält.

12. Verfahren zur Herstellung eines Arzneimittels zur oralen Verabreichung des Wirkstoffs 5-(4-Chlorphenyl)-1-(2,4-dichlorphenyl)-4-methyl-N-1-piperidinyl-1 H-pyrazol-3-carboxamid oder eines Salzes oder Solvats davon oder eines Solvats des Salzes des Wirkstoffs, **dadurch gekennzeichnet, dass** 0,5 bis 50 Gew.-% an Wirkstoff, mindestens ein Bindemittel, mindestens ein Sprengmittel und gegebenenfalls weitere pharmazeutische Hilfsstoffe trocken gemischt und gegebenenfalls trockengranuliert oder direktverpresst werden, wobei das Verfahren keine Nassgranulation umfasst.

13. Verfahren nach Anspruch 12, **dadurch gekennzeichnet, dass** der Wirkstoff als Hydrochloridsalz oder als Solvat des Hydrochloridsalzes eingesetzt wird.

14. Verfahren nach Anspruch 12 oder 13, **dadurch gekennzeichnet, dass** das Arzneimittel entweder kein Netzmittel enthält oder ein von einem Alkylsulfat verschiedenes Netzmittel enthält.

15. Verfahren nach Anspruch 14, **dadurch gekennzeichnet, dass** das Arzneimittel ein oder mehrere Netzmittel enthält, ausgewählt aus Salzen der Gallensäuren, Acylsarkosinen, Polysorbaten, Polyoxyethylenfettalkoholethern, Polyethylenglykolen und synthetischen Blockcopolymeren aus Ethylenoxid und Propylenoxid.

16. Verfahren nach einem der Ansprüche 12 bis 15, **dadurch gekennzeichnet, dass** der Wirkstoff oder das Salz davon oder das Solvat davon oder das Solvat des Salzes des Wirkstoffs in mikronisierter Form vorliegt.

17. Verfahren nach Anspruch 16, **dadurch gekennzeichnet, dass** der Wirkstoff oder das Salz oder Solvat davon oder das Solvat des Salzes des Wirkstoffs eine mittlere Korngröße von zumindest 2,5 µm aufweist.

18. Verfahren nach einem der Ansprüche 12 bis 17, **dadurch gekennzeichnet, dass** das Arzneimittel einen Gehalt an Sprengmittel von 0,1 bis 2,5 Gew.-% aufweist, bezogen auf das Gesamtgewicht des Arzneimittels.

19. Verfahren nach einem der Ansprüche 12 bis 18, **dadurch gekennzeichnet, dass** es sich bei dem Arzneimittel um eine Tablette handelt.

20. Verfahren nach Anspruch 19, **dadurch gekennzeichnet, dass** es den Verfahrensschritt des Direktverpressens umfasst.
